# EUROPEAN PATENT SPECIFICATION

(11) **EP 4 093 346 B1**
(45) Date of publication and mention of the grant of the patent: **21.02.2024**
(21) Application number: 21703508.8
(22) Date of filing: 25.01.2021
(51) Int. Cl.: A61F 5/448

(54) **AN OSTOMY APPLIANCE**
KÜNSTLICHER DARMAUSGANG
ACCESSOIRE POUR STOMIE

(30) Priority: 24.01.2020 GB 202001042
(43) Date of publication of application: 30.11.2022
(73) Proprietor: Salts Healthcare Limited, West Midlands B7 4AA (GB)
(72) Inventor: HOWARD, Lee, Birminghm West Midlands B7 4AA (GB)
(74) Representative: Forresters IP LLP
(86) International application number: PCT/GB2021/050160
(87) International publication number: WO 2021/148815

(56) References cited:
- WO-A1-2017/067559
- CA-A1- 2 215 175
- US-A- 5 257 981
- US-A1- 2016 058 604
- US-A1- 2018 098 876
- US-B1- 6 537 261

## Description

The present invention relates to an ostomy appliance. The invention is defined in the claims.

Ostomy appliances are well known medical devices. Typically, an ostomy appliance has a collecting volume that is placed in fluid communication with a patient's stoma to collect body waste. In more detail, the ostomy appliance includes a first (body-side) wall and a second wall (there may be other walls), which are connected (directly or indirectly) to each other at or near their peripheries to form a collecting volume. The first wall includes a stoma-receiving opening and is connected to a connection member which surrounds the stoma-receiving opening, so that the ostomy appliance can be attached to a patient.

CA2215175 discloses an adhesive wafer for an ostomy pouch, and the combination of such a wafer and pouch, in which the adhesive layer of the wafer is composed of a hydrocolloid-containing skin barrier material and is contoured to provide a relatively thick body portion surrounded by a relatively thin peripheral portion. US5257981 discloses an ostomy appliance for attachment around an aperture of the human body including a plate-like member having an opening extending therethrough. US2018/0098876 discloses an ostomy appliance which may include a wafer base plate to be coupled to a stoma of a patient and having a base layer with an opening positioned inwardly adjacent edges of the base layer, and first fastening pads carried by the base layer. WO2017067559 discloses a coupling system for an ostomy appliance comprising a sealing wafer and a collecting bag.

The present invention aims to provide an improved ostomy appliance.

According to an aspect of the invention we provide an ostomy appliance including: first and second walls which are connected at or near their peripheries to provide a waste collecting cavity therebetween, a stoma-receiving opening provided in the first wall, and a connection member, which member has one or more slits that extend radially outwards relative to the stoma-receiving opening, and which provides for connection to a patient around a stoma, which is connected, directly or indirectly, to the first wall by a connection arrangement which includes: a first connection formation, which extends around the stoma receiving opening and has a periphery and provides support between the connection member and the first wall, and a second connection formation, which is disposed outside of the periphery of the first connection formation and further away from the stoma-receiving opening than the first connection formation.

Further features relating to aspects of the invention are recited by the appended claims.

Some embodiments of the present invention are described below with reference to the enclosed figures, of which:
Figure 1 is a cross-sectional view of an ostomy appliance,
Figures 2, 3A, 3B, 4A, 4B, 5, 6, 7, 8A, 8B, 9A and 9B are examples of connection members
Figure 10 is illustrates a rear (or body) side view of an ostomy appliance.

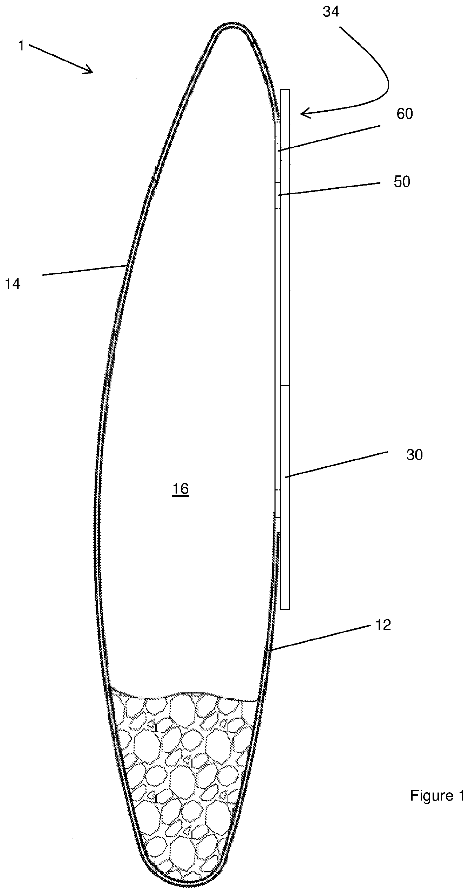

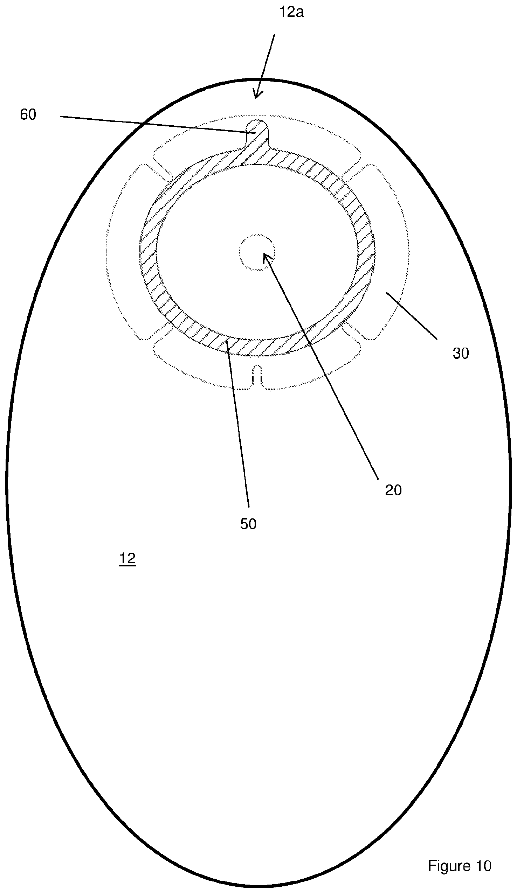

Figures 1 and 10 illustrate an ostomy appliance 1. The ostomy appliance 1 includes a pouch, which is formed from a first wall 12 and a second wall 14 connected at or near their peripheries. A collecting volume 16 for collecting body waste is defined between the first wall 12 and the second wall 14.

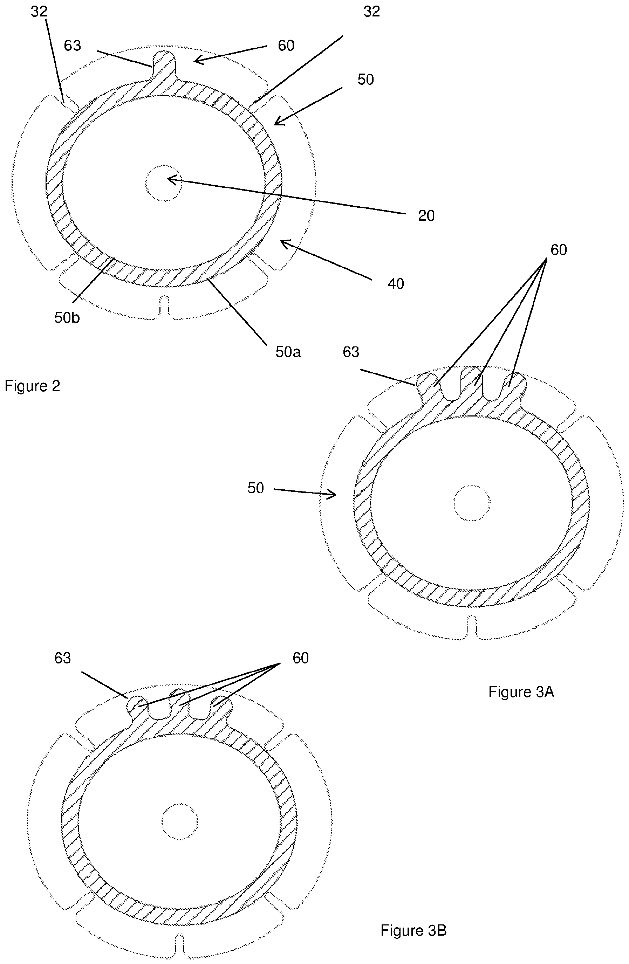

A stoma-receiving opening 20 is provided in the first wall 12 (in the examples illustrated in figures 2 onwards, the stoma-receiving opening 20 is generally circular). The stoma-receiving opening 20 is sized to fit around the patient's stoma. It should be appreciated that the size and shape of the stoma-receiving opening 20 may be adapted to suit an individual patient (and may, for example, be alterable by a patient or medical professional to tailor the ostomy appliance 1 to a specific user).

The ostomy appliance 1 includes a connection member 30. The connection member 30 has a surface 34 which provides a connection to a patient. The connection member 30 is located around the stoma-receiving opening 20, so that the surface 34 is located to connect the ostomy appliance 1 to the patient and provide a seal between the body and the appliance (i.e. to avoid waste being permitted to leak from the collecting volume 16). The surface 34 has a hydrocolloid to adhere the connection member 30 to the skin. However, the surface 34 may connect to the patient with an alternative mechanism, such as, another adhesive, a silicon surface or magnetic connection, for example.

The connection member 30 is connected, either directly or indirectly, to the first wall 12 by a connection arrangement 40. The connection arrangement 40 includes a first connection formation 50 and a second connection formation 60.

Referring to figure 10 specifically, the rear / body side of the ostomy appliance 1 is shown. As can be seen, the connection member 30 and the stoma-receiving opening 20 are disposed towards the top part 12a of the first wall 12. Various examples of the connection arrangement 40 are shown in figures 2 through to 9. Where the same features are shown in multiple embodiments/figures the feature is given the same reference number and where a feature is not exactly the same put is analogous, the reference is the same with a prime (`) added - for example 62 would become 62'. Unless explicitly stated otherwise, all features can be combined with one another as desired.

In some embodiments, the connection member 30 has one or more slits 32 that extend radially outwards from the stoma-receiving opening 20 (in the illustrated embodiments, the connection member 30 has five radially extending slits 32). In this example, each slit 32 extends radially away from the stoma-receiving opening 20 to an outer edge / periphery of the connection member 30. In some embodiments, the connection member 30 has a pair of slits 32 that are spaced apart and angled away from each other. Each one of the pair of slits 32 is positioned to each side and above the stoma-receiving opening 20 (e.g. towards an upper part 12a of the first wall 12. It should be appreciated that the connection member 30 may have fewer or more slits, as desired.

The first connection formation 50 extends around the stoma-receiving opening 20 and has a periphery 50a. The periphery 50a defines an outer edge of the first connection formation 50. In use, the first connection formation 50 provides support between the connection member 30 and the pouch.

In the illustrated examples, the first connection formation 50 extends completely around the stoma-receiving opening 20. Thus, the periphery 50a of the first connection formation 50 also extends completely around the stoma-receiving opening 20. In some embodiments, the first connection formation 50 is substantially symmetrical about an axis that is perpendicular to and substantially central of the connection member. In the illustrated embodiments, the periphery 50a of the first connection formation 50 is generally circular. However, it should be appreciated that the periphery may be another shape as desired (for example, a triangle or square or pentagon or an oval shape which, for example, is flattened above and below the stoma-receiving opening and wider at the sides.

In some embodiments (such as those in figures 2 to 7), the first connection formation 50 includes a second, inner, periphery 50b (which is located radially inside of the periphery 50a). In other words, the second periphery 50b defines the radially inner extent of the attachment. The (first / outer) periphery 50a and the second periphery 50b define a ring which defines the edges of the attachment between the first wall 12 and the connection member 30 (either by a direct or indirect connection - which is explained in more detail below). In this embodiment, the inner periphery 50b is not coincident with the stoma-receiving opening 20 and is, therefore, spaced or distal from the stoma-receiving opening 20. In some embodiments, the first connection formation 50 forms an annular shape.

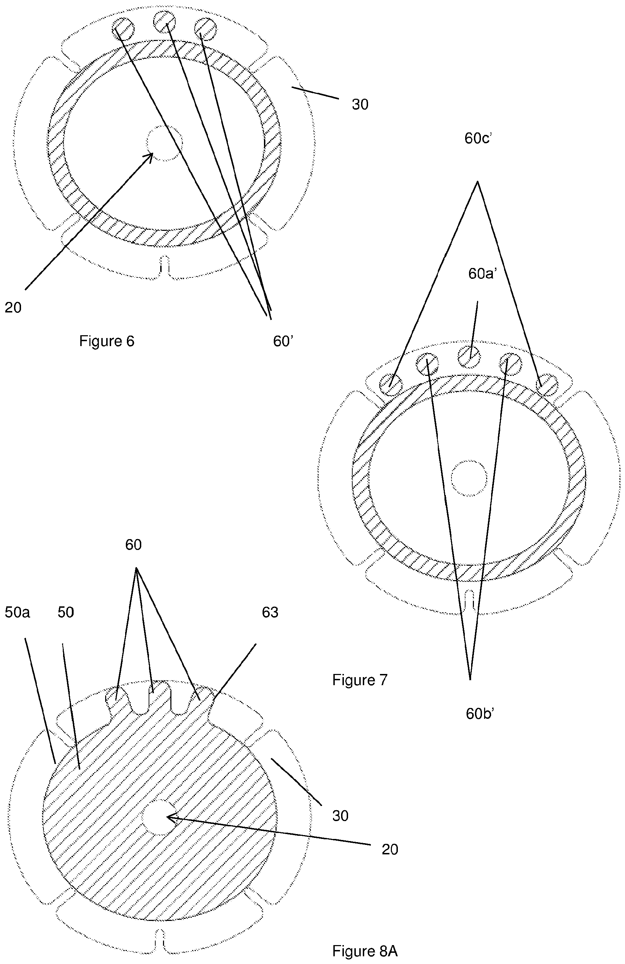

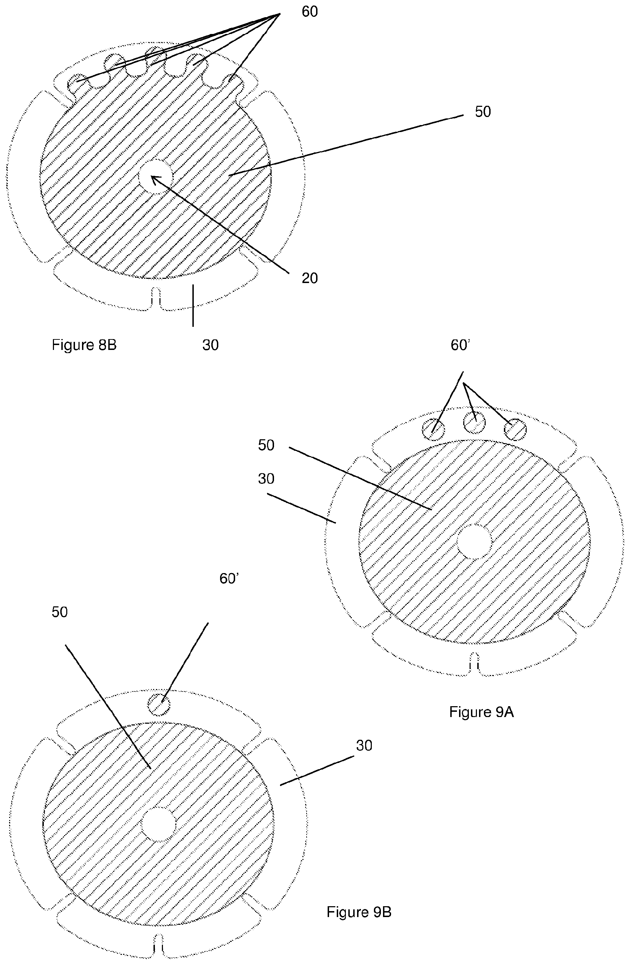

In another example (such as in figures 8A, 8B, 9A and 9B), the first connection formation 50 extends from the (outer) periphery 50a to the inner periphery 50b which is coincident with the stoma-receiving opening 20. In other words, the periphery 50a and the stoma-receiving opening 20 define the area of the attachment covered by the first connection formation 50. Thus, the first connection formation 50 forms a substantially circular attachment between the first wall 12 and the connection member 30 (directly or indirectly) with the stoma-receiving opening 20 in the centre.

The ostomy appliance 1 includes the connection arrangement 40 which connects the first wall 12 to the connection member 30 either directly or indirectly.

A direct connection is a connection that extends between the first wall 12 and the connection member 30. In some embodiments, this may be achieved using a welding process between the material of the first wall 12 and the connection member 30.

An indirect connection allows for a second member between the first wall 12 and the connection member 30. A convex or concave member (not shown) may be positioned around the stoma-receiving opening 20, behind the connection member 30 (i.e. sandwiched between the first wall 12 and the connection member 30). The convex member may support the body tissue around the stoma, so as to improve the connection between the surface 34 and the patient. In such an embodiments, the first connection formation 50 may be formed, firstly, between the first wall 12 and the convex member and, secondly, between the convex member and the connection member 30. In other words, the convex / concave member is connected to the connection member 30, and the first connection formation directly connects the convex member to the first wall. Therefore the connection is an indirect one between the first wall 12 and the connection member 30. It should be appreciated that not all ostomy appliances include the convex member and its use will be dependent on the patient receiving medical assistance.

In the case of an indirect connection, the first connection formation 50 may be formed/provided using an adhesive between the first wall 12 and the convex member and between the convex member and the connection member 30.

The second connection formation 60 is disposed outside of the periphery 50a of the first connection formation 50 and further from the stoma-receiving opening 20 than the first connection formation 50. The second connection formation 60 is located towards an upper part 12a of the first wall 12 (in use, this is above the stoma-receiving opening 20). A function of including a second connection formation 60 is to reduce the effect of the pouch being pulled forwards, away from the connection member 30, as the collecting volume 16 fills with waste. The second connection formation 60 provides an additional anchor between the connection member 30 and the first wall 12.

In some embodiments (for example, figure 2), the second connection formation 60 connects to the first connection formation 50. In the illustrated example in figure 2, the second connection formation 60 extends generally towards to the stoma-receiving opening 20 and connects to the periphery 50a of the first connection formation 50.

It should be appreciated that in this description the stoma-receiving opening 20 is used as a reference point. The stoma-receiving opening 20 is generally positioned substantially centrally in the connection member 30 (and may be cut out from the connection member 30 after manufacture). Thus, it should be appreciated that references to the stoma-receiving opening 20 should also be considered to be references to the substantially central position that the stoma-receiving opening would take in the connection member 30.

The second connection formation 60 has a periphery 63, which defines the outer edge of the second connection formation 60. In some embodiments, the second connection formation 60 includes a base portion that connects to the periphery 50a of the first connection formation 50, and extends away from the stoma-receiving opening 20 to a distal curved end portion. In other words, the periphery 63 of the second connection formation 60 includes a curved portion at a top (i.e. furthest from the stoma-receiving opening 20 and towards the upper part 12a of the first wall 12).

In some embodiments (see for example, figure 3A and 8A), the second connection formation 60 extends to the outer edge of the connection member 30. In other words, the periphery 63 of the second connection formation 60 extends substantially to match the outer periphery of the connection member 30.

In some embodiments (see for example, figure 3B), the second connection formation 60 extends to a location that is spaced from the outer edge of the connection member 30. In other words, the periphery 63 of the second connection formation 60 extends substantially to a location that is spaced from the outer periphery of the connection member 30.

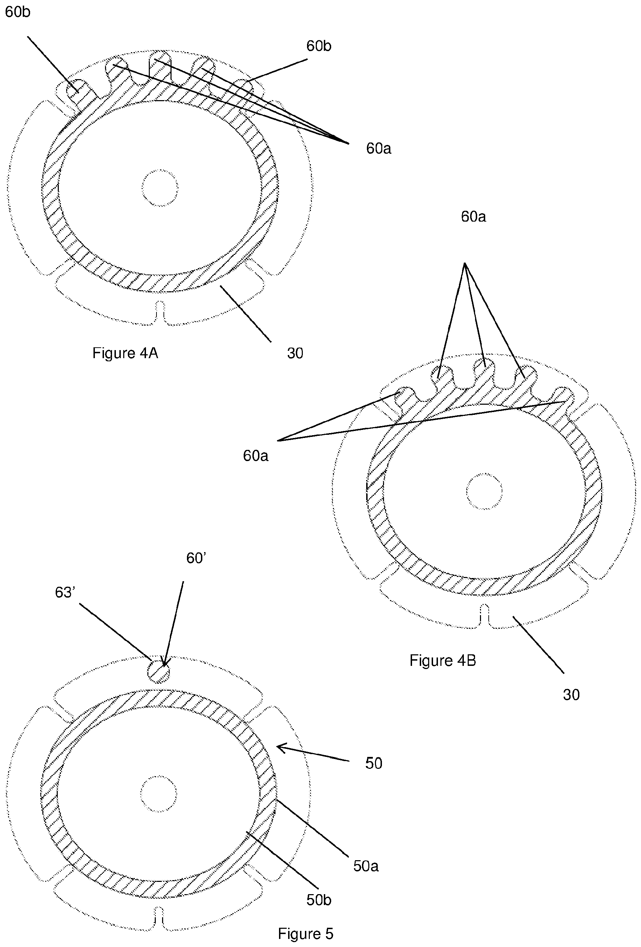

In some embodiments, the connection arrangment 40 includes a plurality of second connection formations 60. For example, figure 3A, 3B and 8A illustrate three separate second connection formations 60 (adjacent each other) that connect to the periphery 50a of the first connection formation 50 at their respective base portions. Figures 4A, 4B and 8B illustrate five second connection formations 60 that are similarly arranged adjacent each other with their base portions connected to the periphery 50a of the first connection formation 50.

Therefore, the second connection formations 60 may each be arranged at a different angle relative to the stoma-receiving opening 20 and extend generally towards the stoma-receiving opening 20. In other words, the second connection formations 60 form a fan arrangement between the periphery 50a of the first connection formation 50 and the top of the connection member 30.

It should be appreciated that each second connection formation 60 may extend different distances from the periphery 50a. For example, the embodiment illustrated in figure 4A includes three "central" second connection formations 60a, all of which extend to the outer periphery of the connection member 30 and two "outer" second connection formations 60b, which are spaced from the edge of the connection ember 30. A similar arrangement of second connection formations 60a, 62b is shown in figure 4B. In this case, the three central second connection formations 60a are spaced from the edge of the connection ember 30 and the two outer second connection formations 60b are shorter still.

Thus, each second connection formation 60 may end at more than one radial distance / different radial distances from the stoma-receiving opening 20 (and/or from the periphery 50a of the first connection formation 50).

In some embodiments (for example, figure 5), the second connection formation 60' is discrete from the first connection formation 50. In the illustrated example in figure 5, there is a single second connection formation 60' which is separate and independent from the first connection formation 50 (and its periphery 50a).

The second connection formation 60' has a periphery 63', which defines the outer edge of the second connection formation 60'. In the illustrated example, the periphery 63' forms a continuous boundary (which in this example is substantially circular). In other words, the second connection formation 60' is substantially circular. It should be appreciated the second connection formation 60' could be a different shape such as, oval, square or rectangular, while still being separate and independent from the first connection formation 50.

Figures 6 and 9A both illustrate a connection arrangement 40 that has two second connection formations 60', and figure 7 illustrates five second connection formations 60'. In each of these examples, each second connection formations 60' is separate from and adjacent the next second connection formation 60'.

In both examples, a second connection formation 60' is substantially centred about an axis that extends vertically from the top of the first wall 12 downwardly and through the stoma-receiving opening 20. The further second connection formations 60' are located either side. In this example, the second connection formations 60' are symmetrical about the central axis - i.e. in the group of three second connection formations 60' there is one second connection formation 60' placed either side of the central second connection formation 60' and for the groups of five second connection formations 60', there are two either side of the central formation 60'.

It should also be appreciated that the second connection formations 60' may be different radial distances from the stoma-receiving opening 20 (and/or from the periphery 50a of the first connection formation 50). For example, figure 7 illustrates a first central second connection formation 60a' positioned a first (radial) distance from the stoma-receiving opening 20 (and periphery 50a). There are two middle second connection formations 60b', positioned either side of the first second connection formation 60a', and positioned a second shorter distance (i.e. a second, radial distance) from the stoma receiving opening 20. Further, there are two outer second connection formations 60c', positioned either side of the middle second connection formations 60b', and are positioned at a third shorter distance (i.e. a third radial distance) from the stoma-receiving opening 20.

It should also be appreciated that in an embodiment in which there are multiple second connection formations 60, 62', both types of second connection formation 60, 62' may be included. In other words, second connection formations 60 that connect to the first connection formation 50 may be mixed with second connection formations 60' that are discrete from the first connection formation 50.

In some embodiments (such as those illustrated in the figures) where the connection member 30 includes one or more slits 32, the or each slit 32 has a radially innermost end which defines a radial distance from the stoma-receiving opening 20. The periphery 50a of the first connection formation 50 is disposed either at this radial distance or a smaller radial distance from the stoma-receiving opening 20. In other words, the first connection formation 50 is located within the radially innermost end of the slits 32.

The second connection formation 60 is located either at substantially the same radial distance or a larger radial position than that defined by the inner most ends of the slit(s) 32.

In some embodiments, even if the first connection formation 50 is provided indirectly (as described above), the second connection formation 60 may still be provided as a direct connection between the connection member 30 and the first wall 12.

The advantage of having a second connection formation 60, 62' means that additional support can be provided between the first wall 12 (specifically the top part 12a of the first wall 12) and the connection member 30 (which is above where the existing support was provided previously). Previously, as the collecting volume 16 fills during use, the top of the ostomy appliance 1 sags forward under the weight pulling at the bottom. The additional connection(s) provided by the second connection formation(s) 60, 60' prevents and/or reduces sagging of the pouch because the first wall 12 is attached to the connection member 30 at a higher position and, therefore, the first wall 12 is prevented from pulling forward away from the connection member 30 (and the body the appliance 1 is attached to).

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

Although certain example embodiments of the invention have been described, the scope of the appended claims is not intended to be limited solely to these embodiments. The scope of the invention is defined by the appended claims.

## Claims

1. An ostomy appliance (1) including:
first (12) and second (14) walls which are connected at or near their peripheries to provide a waste collecting cavity therebetween,
a stoma-receiving opening (20) provided in the first wall (12), and
a connection member (30), which has one or more slits (32) that extend radially outwards relative to the stoma-receiving opening (20), and which provides for connection to a patient around a stoma, which is connected, directly or indirectly, to the first wall (12) by a connection arrangement (40) which includes:
a first connection formation (50), which extends around the stoma receiving opening (20) and has a periphery (50a) and provides support between the connection member (30) and the first wall (12), and
a second connection formation (60), which is disposed outside of the periphery (50a) of the first connection formation (50) and further away from the stoma-receiving opening (20) than the first connection formation (50) and at the same radial distance or a larger radial position from the stoma-receiving opening (20) than that defined by an inner most end of the one or more slits (32).

2. An ostomy appliance (1) according to claim 1 wherein the second connection formation (60) is positioned, in use, above the stoma-receiving opening (20).

3. An ostomy appliance (1) according to any of the preceding claims wherein the second connection formation (60) is discrete from the first connection formation (50), and optionally wherein the second connection formation (60) is substantially circular.

4. An ostomy appliance (1) according to claim 1 or 2 wherein the second connection formation (60) is connected to the first connection formation (50).

5. An ostomy appliance (1) according to claim 4 wherein the second connection formation (60) extends generally towards the stoma-receiving opening (20) and connects to the periphery (50a) of the first connection zone.

6. An ostomy appliance (1) according to claims 4 or 5 wherein the second connection formation (60) includes a curved end portion positioned distally from the first connection formation (50).

7. An ostomy appliance (1) according to any of the preceding claims wherein the attachment zone of the second connection formation (60) extends to an edge of the connection member (30) or extends to a position which is spaced from an edge of the connection member (30).

8. An ostomy appliance (1) according to any of the preceding claims wherein there is a plurality of second connection formations (60) and optionally wherein each of the second connection formations (60) is either discrete from the first connection formation (50) or connected to the first connection formation (50).

9. An ostomy appliance (1) according to claim 8 wherein there are between two and five second connection formations (60), which are spaced apart from each other.

10. An ostomy appliance (1) according to claim any of the preceding claims wherein the connection member (30) has a pair of slits (32) which are spaced apart and angled away from each other above the stoma-receiving opening (20), towards an upper part of the first wall (12), and wherein the second connection formation (60) is positioned between the pair of slits (32).

11. An ostomy appliance according to any of the preceding claims wherein the or each slit (32) has a radially innermost end which is spaced at a radial distance from a centre of the stoma-receiving opening (20) and the periphery (50a) of the first connection formation (50) is disposed either at this or a smaller radial distance from the centre of the stoma-receiving opening (20).

12. An ostomy appliance (1) according to any of the preceding claims wherein the first connection formation (50) extends completely around the stoma-receiving opening (20).

13. An ostomy appliance (1) according to claim 11 wherein the first connection formation (50) has a second, inner, periphery (50b) which is either coincident with the stoma-receiving opening (20) or spaced radially outwardly from the stoma-receiving opening (20).

14. An ostomy appliance (1) according to any of the preceding claims wherein the first connection formation (50) is substantially symmetrical about a midline which, in use, extends substantially vertically through the stoma-receiving opening (20) and/or wherein the first connection formation (50) is substantially circular or annular.

15. An ostomy appliance (1) according to any of the preceding claims which further includes a convex or concave support member, which is positioned between the first wall (12) and the connection member (30) and connected to the latter, and wherein the first connection formation (50) directly connects the convex member to the first wall (12) and/or wherein the second connection formation (60) provides a direct connection between the first wall (12) and the connection member (30).

## Patentansprüche

1. Künstlicher Darmausgang (1), beinhaltend:
eine erste (12) und eine zweite (14) Wand, die an oder nahe ihrer Peripherien angeschlossen sind, um einen Ausscheidungssammelhohlraum dazwischen bereitzustellen,
eine Stoma-Aufnahmeöffnung (20), die in der ersten Wand (12) bereitgestellt ist, und
ein Anschlusselement (30), das einen oder mehrere Schlitze (32) aufweist, die sich relativ zu der Stoma-Aufnahmeöffnung (20) radial nach außen erstrecken, und das für einen Anschluss an einen Patienten um ein Stoma herum sorgt, das direkt oder indirekt an die erste Wand (12) durch eine Anschlussanordnung (40) angeschlossen ist, die Folgendes beinhaltet:
eine erste Anschlussformation (50), die sich um die Stoma-Aufnahmeöffnung (20) herum erstreckt und eine Peripherie (50a) aufweist und für eine Abstützung zwischen dem Anschlusselement (30) und der ersten Wand (12) sorgt, und
eine zweite Anschlussformation (60), die außerhalb der Peripherie (50a) der ersten Anschlussformation (50) und weiter weg von der Stoma-Aufnahmeöffnung (20) als die erste Anschlussformation (50) und in dem gleichen radialen Abstand oder einer größeren radialen Position von der Stoma-Aufnahmeöffnung (20) als diejenige, die von einem innersten Ende des einen oder mehrerer Schlitze (32) definiert ist, angeordnet ist.

2. Künstlicher Darmausgang (1) nach Anspruch 1, wobei die zweite Anschlussformation (60), bei Verwendung, oberhalb der Stoma-Aufnahmeöffnung (20) positioniert ist.

3. Künstlicher Darmausgang (1) nach einem der vorhergehenden Ansprüche, wobei die zweite Anschlussformation (60) von der ersten Anschlussformation (50) getrennt ist, und optional, wobei die zweite Anschlussformation (60) im Wesentlichen kreisförmig ist.

4. Künstlicher Darmausgang (1) nach Anspruch 1 oder 2, wobei die zweite Anschlussformation (60) an die erste Anschlussformation (50) angeschlossen ist.

5. Künstlicher Darmausgang (1) nach Anspruch 4, wobei sich die zweite Anschlussformation (60) im Allgemeinen hin zu der Stoma-Aufnahmeöffnung (20) erstreckt und an die Peripherie (50a) der ersten Anschlusszone angeschlossen ist.

6. Künstlicher Darmausgang (1) nach Ansprüchen 4 oder 5, wobei die zweite Anschlussformation (60) einen gekrümmten Endabschnitt beinhaltet, der distal von der ersten Anschlussformation (50) positioniert ist.

7. Künstlicher Darmausgang (1) nach einem der vorhergehenden Ansprüche, wobei sich die Befestigungszone der zweiten Anschlussformation (60) zu einem Rand des Anschlusselements (30) erstreckt oder sich zu einer Position, die von einem Rand des Anschlusselements (30) beabstandet ist, erstreckt.

8. Künstlicher Darmausgang (1) nach einem der vorhergehenden Ansprüche, wobei es eine Vielzahl von zweiten Anschlussformationen (60) gibt, und optional, wobei jede der zweiten Anschlussformationen (60) entweder von der ersten Anschlussformation (50) getrennt oder an die erste Anschlussformation (50) angeschlossen ist.

9. Künstlicher Darmausgang (1) nach Anspruch 8, wobei es zwischen zwei und fünf zweite Anschlussformationen (60) gibt, die voneinander beabstandet sind.

10. Künstlicher Darmausgang (1) nach einem der vorhergehenden Ansprüche, wobei das Anschlusselement (30) ein Paar von Schlitzen (32) aufweist, die oberhalb der Stoma-Aufnahmeöffnung (20) hin zu einem oberen Teil der ersten Wand (12) voneinander beabstandet und weg gewinkelt sind, und wobei die zweite Anschlussformation (60) zwischen dem Paar von Schlitzen (32) angeordnet ist.

11. Künstlicher Darmausgang nach einem der vorhergehenden Ansprüche, wobei der oder jeder Schlitz (32) ein radial innerstes Ende aufweist, das in einem radialen Abstand von einer Mitte der Stoma-Aufnahmeöffnung (20) beabstandet ist, und die Peripherie (50a) der ersten Anschlussformation (50) entweder in diesem oder einem kleineren radialen Abstand von der Mitte der Stoma-Aufnahmeöffnung (20) angeordnet ist.

12. Künstlicher Darmausgang (1) nach einem der vorhergehenden Ansprüche, wobei sich die erste Anschlussformation (50) vollständig um die Stoma-Aufnahmeöffnung (20) herum erstreckt.

13. Künstlicher Darmausgang (1) nach Anspruch 11, wobei die erste Anschlussformation (50) eine zweite, innere Peripherie (50b) aufweist, die entweder mit der Stoma-Aufnahmeöffnung (20) zusammenfällt oder von der Stoma-Aufnahmeöffnung (20) radial nach außen beabstandet ist.

14. Künstlicher Darmausgang (1) nach einem der vorhergehenden Ansprüche, wobei die erste Anschlussformation (50) im Wesentlichen symmetrisch um eine Mittellinie ist, die sich, bei Verwendung, im Wesentlichen vertikal durch die Stoma-Aufnahmeöffnung (20) erstreckt, und/oder wobei die erste Anschlussformation (50) im Wesentlichen kreisförmig oder ringförmig ist.

15. Künstlicher Darmausgang (1) nach einem der vorhergehenden Ansprüche, der ferner ein konvexes oder konkaves Stützelement beinhaltet, das zwischen der ersten Wand (12) und dem Anschlusselement (30) positioniert und an Letzteres angeschlossen ist, und wobei die erste Anschlussformation (50) das konvexe Element direkt an die erste Wand (12) anschließt und/oder wobei die zweite Anschlussformation (60) einen direkten Anschluss zwischen der ersten Wand (12) und dem Anschlusselement (30) bereitstellt.

## Revendications

1. Appareillage stomique (1), comprenant :
des première (12) et seconde (14) parois qui sont reliées au niveau de leur périphérie ou à proximité de celle-ci pour former une cavité de collecte des déchets entre elles,
une ouverture de réception de stomie (20) pratiquée dans la première paroi (12), et
un élément de liaison (30), qui comporte une ou plusieurs fentes (32) qui s'étendent radialement vers l'extérieur par rapport à l'ouverture de réception de stomie (20) et qui assure une liaison à un patient autour d'une stomie, qui est reliée, directement ou indirectement, à la première paroi (12) par une configuration de liaison (40) qui comprend :
une première formation de liaison (50), qui s'étend autour de l'ouverture de réception de stomie (20) et présente une périphérie (50a) et fournit un support entre l'élément de liaison (30) et la première paroi (12), et
une seconde formation de liaison (60), qui est disposée à l'extérieur de la périphérie (50a) de la première formation de liaison (50) et en outre à l'opposé de l'ouverture de réception de stomie (20) par rapport à la première formation de liaison (50) et à la même distance radiale ou à une position radiale plus grande de l'ouverture de réception de stomie (20) que celle définie par une extrémité la plus intérieure de la ou des fentes (32).

2. Appareillage stomique (1) selon la revendication 1, dans lequel la seconde formation de liaison (60) est positionnée, lors de l'utilisation, au-dessus de l'ouverture de réception de stomie (20).

3. Appareillage stomique (1) selon l'une quelconque des revendications précédentes, dans lequel la seconde formation de liaison (60) est distincte de la première formation de liaison (50), et éventuellement dans lequel la seconde formation de liaison (60) est sensiblement circulaire.

4. Appareillage stomique (1) selon la revendication 1 ou 2, dans lequel la seconde formation de liaison (60) est reliée à la première formation de liaison (50).

5. Appareillage stomique (1) selon la revendication 4, dans lequel la seconde formation de liaison (60) s'étend généralement vers l'ouverture de réception de stomie (20) et se relie à la périphérie (50a) de la première zone de liaison.

6. Appareillage stomique (1) selon la revendication 4 ou 5, dans lequel la seconde formation de liaison (60) comprend une partie d'extrémité incurvée positionnée distale par rapport à la première formation de liaison (50).

7. Appareillage stomique (1) selon l'une quelconque des revendications précédentes, dans lequel la zone de liaison de la seconde formation de liaison (60) s'étend vers un bord de l'élément de liaison (30) ou s'étend vers une position qui est espacée d'un bord de l'élément de liaison (30).

8. Appareillage stomique (1) selon l'une quelconque des revendications précédentes, dans lequel il existe une pluralité de secondes formations de liaison (60) et, éventuellement, dans lequel chacune des secondes formations de liaison (60) est distincte de la première formation de liaison (50) ou reliée à la première formation de liaison (50).

9. Appareillage stomique (1) selon la revendication 8, dans lequel il y a entre deux et cinq secondes formations de liaison (60) qui sont espacées les unes des autres.

10. Appareillage stomique (1) selon l'une quelconque des revendications précédentes, dans lequel l'élément de liaison (30) présente une paire de fentes (32) qui sont espacées et inclinées l'une à l'opposé de l'autre au-dessus de l'ouverture de réception de stomie (20), vers une partie supérieure de la première paroi (12), et dans lequel la seconde formation de liaison (60) est positionnée entre la paire de fentes (32).

11. Appareillage stomique selon l'une quelconque des revendications précédentes, dans lequel la ou chaque fente (32) présente une extrémité radialement la plus intérieure qui est espacée à une distance radiale d'un centre de l'ouverture de réception de stomie (20), et la périphérie (50a) de la première formation de liaison (50) est disposée à cette distance radiale ou à une distance radiale plus petite du centre de l'ouverture de réception de stomie (20).

12. Appareillage stomique (1) selon l'une quelconque des revendications précédentes, dans lequel la première formation de liaison (50) s'étend complètement autour de l'ouverture de réception de stomie (20).

13. Appareillage stomique (1) selon la revendication 11, dans lequel la première formation de liaison (50) présente une seconde périphérie intérieure (50b) qui coïncide avec l'ouverture de réception de stomie (20) ou qui est espacée radialement vers l'extérieur de l'ouverture de réception de stomie (20).

14. Appareillage stomique (1) selon l'une quelconque des revendications précédentes, dans lequel la première formation de liaison (50) est sensiblement symétrique par rapport à une ligne médiane qui, lors de l'utilisation, s'étend sensiblement verticalement à travers l'ouverture de réception de stomie (20) et/ou dans lequel la première formation de liaison (50) est sensiblement circulaire ou annulaire.

15. Appareil stomique (1) selon l'une quelconque des revendications précédentes, comprenant en outre un élément de support convexe ou concave qui est positionné entre la première paroi (12) et l'élément de liaison (30) et qui est relié à ce dernier, et dans lequel la première formation de liaison (50) relie directement l'élément convexe à la première paroi (12) et/ou dans lequel la seconde formation de liaison (60) assure une liaison directe entre la première paroi (12) et l'élément de liaison (30).
